(19) Europäisches Patentamt
European Patent Office
Office européen des brevets

(11) Numéro de publication: **0 040 144**
**B1**

(12) # FASCICULE DE BREVET EUROPEEN

(45) Date de publication du fascicule du brevet: **08.08.84**

(51) Int. Cl.³: **C 08 B 37/10, A 61 K 31/725**

(21) Numéro de dépôt: **81400728.2**

(22) Date de dépôt: **08.05.81**

(54) **Nouveaux polysaccharides sulfatés, procédés pour leur préparation et leur utilisation comme médicaments.**

(30) Priorité: **14.05.80 FR 8010791**

(43) Date de publication de la demande:
**18.11.81 Bulletin 81/46**

(45) Mention de la délivrance du brevet:
**08.08.84 Bulletin 84/32**

(84) Etats contractants désignés:
**BE CH DE FR GB IT LI LU NL SE**

(56) Documents cités:
**EP - A - 0 014 184**
**FR - A - 2 100 735**

**CONNECTIVE TISSUE RESEARCH, vol. 3 (1975) S. HIRANO et al.: "Depolymerization and De-N-Acetylation of Glycosaminoglycuronans by the action of alkali in the presence of sodium borohydride", pages 73-79**
**ADVANCES IN CARBOHYDRATE CHEMISTRY AND BIOCHEMISTRY, Academic Press 1974, vol. 29 New York, US J. KISS: "Beta-Eliminative degradation of carbohydrates containing uronic acid residues", pages 238-245**

**Le dossier contient des informations techniques présentées postérieurement au dépôt de la demande et ne figurant pas dans le présent fascicule.**

(73) Titulaire: **PHARMUKA LABORATOIRES, 35 Quai du Moulin de Cage, F-92231 Gennevilliers (FR)**

(72) Inventeur: **Mardiguian, Jean, 3, Villa Christine, F-94210 La Varenne Saint-Hilaire (FR)**

(74) Mandataire: **Gaumont, Robert et al, RHONE-POULENC RECHERCHES Service Brevets Pharma 25, Quai Paul Doumer, F-92408 Courbevoie Cedex (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**EP 0 040 144 B1**

BUNDESDRUCKEREI BERLIN

## Beschreibung

La présente invention a pour objet de nouveaux mélanges de polysaccharides sulfatés de poids moléculaire moyen en poids inférieur à celui de l'héparine, utilisables comme agents anticoagulants et antithrombotiques dans la prévention et le traitement des thromboses et comme agents hypolipémiants.

L'héparine est un mélange de mucopolysaccharides sulfatés d'origine animale, largement utilisé pour ses propriétés anticoagulantes et antithrombotiques, notamment dans la prévention des thromboses veineuses post-opératoires, et pour ses propriétés hypolipémiantes. On sait qu'elle agit dans le processus de la coagulation en activant un inhibiteur naturel de la coagulation contenu dans le sang, l'antithrombine III. L'activation de cette protéine a pour effet d'inhiber l'action de deux protéases, le facteur X-activé (facteur Xa) d'une part et la thrombine d'autre part.

L'activité anticoagulante in vitro de l'héparine est mesurée communément par les techniques officielles des pharmacopées, en particulier des pharmacopées américaine, anglaise ou française, en se référant à un étalon international. Mais il est maintenant possible de mesurer ses activités spécifiques in vitro, d'une part vis-à-vis du facteur Xa et d'autre part vis-à-vis de la thrombine (cf. par exemple A. TEIEN et al., Thrombosis Research, 11, 107, 1977).

Les héparines commerciales, qui sont des mélanges de polysaccharides dont le poids moléculaire moyen en poids est supérieur à 10 000 daltons et dont la dispersion de poids moléculaires va de 4000 à 45 000 daltons environ, présentent les activités in vitro suivantes:

- Activité anticoagulante mesurée par la méthode de la pharmacopée française, 8ème èdition, monographie héparine, en se référant au 3ème étalon international (ou activité anticoagulante codex) : 140 à 200 u.i./mg.
- Activité anti-Xa mesurée par la méthode de TEIEN et al. (déjà cité), en se référant au 3ème étalon international : 160 à 180 u.i./mg.
- Activité A.P.T.T. (Activated Partial Thromboplastin Time) mesurée par la méthode de TEIEN et al. (déjà cité), en se référant au 3eme étalon international : 150 à 170 u.i./mg.

L'héparine est obligatoirement administrée par voie parentérale (en pratique la voie sous-cutanée) et sa durée d'action est relativement courte, d'où les deux inconvénients majeurs suivants : nécessité d'effectuer trois administrations par jour et fréquence relativement élevée d'accidents hémorragiques post opératoires.

Il est connu (cf. JOHNSON et al., Thrombos. Haemostas. Stuttg., 1976, 35, 586—591; LANE et al, Thrombosis Research 16, 651—662, Pergamon Press Ltd. 1979; LASKER, CHIU, Annals N.Y. Acad. Sci., 222, 1973, 971—977; LASKER, Adv. Exp. Med. Biol. 52, 1975, 119—130) que l'on peut obtenir par fractionnement de l'héparine, par exemple par filtration sur gel Sephadex, des fractions de poids moléculaire moyen plus faible que celui de l'heparine et de dispersion de poids moléculaires moins large que celle de l'héparine. Ainsi que le montrent les essais effectués in vitro et in vivo, de telles fractions d'une part sont relativement plus actives sur le facteur X-activé que sur la thrombine (c'est-à-dire qu'elles présentent un rapport activité anti-Xa nettement supérieur à 1), et d'autre part activité antithrombinique sont plus aisément absorbées dans la circulation à partir d'une injection sous-cutanée que l'héparine elle-même, d'où une activité plasmatique plus élevée et de plus longue durée que celle de l'héparine.

Il est connu également (cf. LASKER et CHIU déjà cité; LASKER déjà cité; brevet U.S. 3 766 167; PERLIN et al, CARBOHYDRATE Research, 18, 1971, 185—194) de préparer, par hydrolyse enzymatique de l'héparine, des produits de dépolymérisation ayant un poids moléculaire moyen bas (pratiquement 5300 à 4500 daltons, déterminé par ultracentrifugation) et une activité anticoagulante, déterminée par la méthode U.S.P. N° XVII, d'environ 70 u.i./mg. Ces produits de dépolymérisation peuvent être fractionnés, par filtration sur gel Sephadex, en fractions dont les poids moléculaires moyens, déterminés par ultracentrifugation, vont de 3200 à 5900 daltons et les activités anticoagulantes de 45 à 95 u.i./mg (méthode U.S.P.). Ces produits de dépolymérisation et les fractions qui en sont issues sont actifs par la voie orale aussi bien que par la voie parentérale.

Il est connu aussi (cf. LASKER déjà cité) de préparer, par dépolymérisation de l'héparine à l'aide d'acide ascorbique et d'eau oxygénée, des produits de bas poids moléculaire moyen. La dépolymérisation à l'aide d'acide ascorbique et d'eau oxygénée conduit, après fractionnement à l'alcool, à des fractions ayant un poids moléculaire moyen de 4000 à 7100 daltons et une activité anticoagulante de 12 à 100 u.i./mg (méthode U.S.P.).

Enfin, il est connu (cf. demande de brevet anglais 2 002 406 A publiée le 21.02.1979) de préparer, par resulfatation de produits de dépolymérisation de l'héparine dénués d'activité anticoagulante, des oligopolysaccharides ayant un poids moléculaire moyen, déterminé par la méthode de Somogy, de 2600 à 5500 daltons, un pouvoir rotatoire spécifique mesuré en solution aqueuse à 20°C de +40° à +50° et une activité anticoagulante inférieure à 50 u.i./mg (méthode U.S.P.) qui seraient actifs aussi bien par voie orale que par voie parentérale pour la prévention des thromboses.

Les mélanges de polysaccharides sulfatés selon l'invention sont des mélanges de polysaccharides sulfatés ayant la structure générale des polysaccharides constitutifs de l'héparine, c'est-à-dire présentant les mêmes chaînons élémentaires que les polysaccharides constitutifs de l'héparine, lesdits chaînons élémentaires étant liés de la même manière que dans ces derniers. Toutefois, contrairement aux polysaccharides constitutifs de l'héparine, les polysaccharides constitutifs des mélanges selon l'invention comportent une double liaison éthylénique à l'une des extrémités de leur chaîne.

Dans les mélanges selon l'invention, les groupes acides des polysaccharides sulfatés peuvent être sous forme libre ou sous forme salifiée, en particulier sous forme de sel de sodium, de calcium ou de magnésium.

Les mélanges selon l'invention présentent, à l'état de sel de sodium, les caractéristiques suivantes:

- pourcentage de soufre: 9% à 13,5%
- pourcentage d'azote: 1,8% à 2,5%
- pourcentage d'acides uroniques: 20% à 30%
- poids moléculaire moyen en poids: 2000 à 10 000 daltons
- pouvoir rotatoire spécifique en solution aqueuse à 20°C: $[\alpha]_D^{20} = +25° à +55°$

Les teneurs en soufre et en azote et le pouvoir rotatoire indiqués ci-dessus ont été déterminés par les méthodes de la pharmacopée française, 8ème édition, monographie héparine. La teneur en acides uroniques a été déterminée par la méthode de N. BLUMENKRANTZ et al. (Analytical Biochemistry, 54, 484, 1973). Le poids moléculaire moyen en poids a été déterminé par chromatographie par perméation de gel sur gel de polyacrylamide-agarose en se référant à des étalons constitués par des héparines de poids moléculaire moyen connu, selon la méthode de Andersson et al. (Thrombosis Research 9, 575, 1976).

Les polysaccharides constitutifs des mélanges selon l'invention répondent en particulier à la formule:

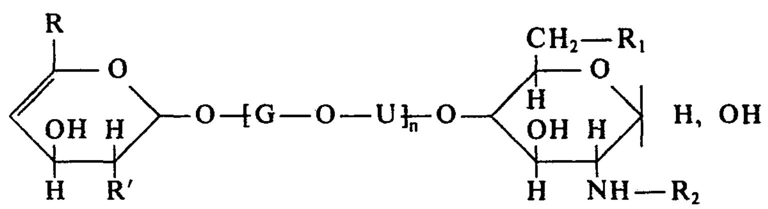 (I)

dans laquelle R désigne un atome d'hydrogène ou un groupe carboxylique, sous forme acide ou sous forme salifiée, R' désigne un groupe OH ou un groupe sulfate, sous forme acide ou sous forme salifiée, $R_1$ désigne un groupe OH ou un groupe sulfate, sous forme acide ou sous forme salifiée, $R_2$ désigne un groupe sulfonate, sous forme acide ou sous forme salifiée, ou un groupe acétyle, —O— désigne un pont oxygène, les chaînons G désignent les chaînons du type glucosamine figurant dans la structure de l'héparine, les chaînons U désignent les chaînons du type acide uronique (acide D-glucuronique, acide L-iduronique, acide L-iduronique sulfaté) figurant dans la structure de l'héparine, et n est un nombre entier de 3 à 20.

Dans la formule (I) ci-dessus, les groupes acides des polysaccharides peuvent être sous forme libre ou sous forme salifiée, en particulier sous forme de sel de sodium, de calcium ou de magnésium.

Bien que la présente invention concerne l'ensemble des mélanges tels que définis précédemment, elle a plus particulièrement pour objet ceux de ces mélanges qui, à l'état de sel de sodium, présentent les caractéristiques additionnelles indiquées pour les catégories I, II, III dans le tableau A ci-après:

Tableau A

| | Catégorie I | Catégorie II | Catégorie III |
|---|---|---|---|
| Poids moléculaire moyen en poids (en daltons) | 8000 à 10 000 | 3000 à 8000 | 2000 à 7000 |
| Activité anticoagulante in vitro Codex | 130 à 160 | 80 à 140 | 10 à 80 |
| Activité in vitro anti-Xa | 130 à 180 | 120 à 250 | 80 à 250 |
| Activité in vitro A.P.T.T. | 100 à 150 | 80 à 120 | 10 à 80 |
| $\frac{\text{Rapport activité in vitro anti-Xa}}{\text{activité in vitro A.P.T.T.}}$ | 1 à 1,5 | 1,4 à 3 | 2 à 10 |

L'activité anticoagulante Codex indiquée dans le tableau A a été déterminée par la méthode de la pharmacopée française, 8ème édition, monographie héparine. Les activités anti-Xa et A.P.T.T. indiquées dans le tableau A de même que celles indiquées plus loin dans les tableaux B et C ont été déterminées par la méthode de TEIEN et al. (déjà cité), en suivant les modes opératoires décrits ci-dessous:

a) Mesure de l'activité anti-Xa

Cette activité est déterminée sur plasma de bovin déplaquetté à l'aide du substrat chromogène S 2222 [peptide chromogène de structure (N-benzoyl) Ile-Glu-Gly-Arg-(p-nitro)anilide] et en se référant au 3ème étalon international.

A 100 µl de solution du produit à tester ou de l'étalon en milieu tampon aqueux Tris/EDTA pH 8,4, représentant 0,02 à 0,08 µg de produit ou d'étalon, on ajoute 100 µl de plasma de bovin citraté, préalablement dilué au 2/5 avec le tampon tris/EDTA précédent. Après trois minutes d'incubation à 37°C, on ajoute 100 µl d'une solution aqueuse de facteur Xa bovin représentant 7 n Kat de facteur Xa. Après 30 secondes d'incubation on ajoute 200 µl de solution aqueuse de S 2222 représentant 0,2 µmole de S 2222. Après trois minutes d'incubation on ajoute 300 µl d'acide acétique et on lit la densité optique de la solution à 405 nm par rapport à de l'eau distillée.

En traçant les courbes qui donnent la densité optique mesurée en fonction de la concentration d'une part dans le cas du produit à tester, d'autre part dans le cas de l'étalon, on obtient deux droites, l'une relative au produit à tester l'autre relative à l'étalon. L'activité du produit à tester, exprimée en unités internationales par mg, est donnée par la formule:

$$173\ \frac{\text{pente de la droite relative au produit à tester}}{\text{pente de la droite relative à l'étalon}}$$

le chiffre 173 correspondant à la valeur de l'activité pour le 3ème étalon international. Le rapport ci-dessus des pentes des deux droites est égal à l'inverse du rapport des concentrations en produit à tester et en étalon conduisant à une même densité optique.

b) Mesure de l'activité A.P.T.T.

Le produit à tester et le 3ème étalon international sont dissous dans une solution aqueuse 0,15 M de chlorure de sodium, puis dilués avec du plasma de bovin citraté déplaquetté de façon à obtenir des concentrations en produit à tester (ou en étalon) de 0 à 4 µg/ml.

A 100 µl de la solution ainsi réalisée on ajoute 100 µl de réactif »automated APTT Precibio« (réactif à base de de phospholipides de cerveau de lapin et de silice micronisée) et, après une incubation de 5 minutes à 37°C, 100 µl d'une solution aqueuse 0,025 M de chlorure de calcium. On mesure le temps de coagulation à l'aide d'un fibromètre Bio-Merieux.

En traçant les courbes qui donnent le logarithme du temps de coagulation en fonction de la concentration d'une part dans le cas du produit à tester, d'autre part dans le cas de l'étalon, on obtient deux droites. L'activité du produit à tester, exprimée en unités internationales par mg, est donnée par la formule:

$$173\ \frac{\text{pente de la droite relative au produit à tester}}{\text{pente de la droite relative à l'étalon}}$$

le chiffre 173 correspondant à la valeur de l'activité pour le 3ème étalon international.

Toutes les activités figurant dans les tableaux A, B et C sont exprimées en unités internationales par mg (u.i./mg), en se référant au 3ème étalon international.

Les mélanges selon l'invention sont préparés par action d'une base minérale ou organique sur un ester d'héparine résultant de l'estérification partielle ou totale des groupes acide carboxylique de l'héparine. Dans cet ester les groupes acides de l'héparine non estérifiés (c'est-à-dire les groupes sulfate acide et éventuellement une partie des groupes acide carboxylique) peuvent être à l'état libre ou sous forme de sel, en particulier de sel alcalin tel que le sel de sodium, de sel alcalino-terreux tel que le sel de calcium, de sel de magnésium ou de sel d'ammonium quaternaire à longue chaîne tel que le sel de benzéthonium.

Dans le cas où l'ester d'héparine est soluble dans l'eau (cas par exemple où les groupes acides non estérifiés sont sous forme de sel de sodium), la réaction entre l'ester et la base peut être effectuée dans l'eau, à une température de 20°C à 80°C, la concentration molaire de la base dans le milieu étant de préférence comprise entre 0,1 et 0,6. Comme bases utilisables on peut citer les bases solubles dans l'eau et en particulier l'hydroxyde de sodium, l'hydroxyde de potassium, les carbonates alcalins, la triéthylamine, la triéthylènediamine, la quinuclidine, le diaza-1,5 bicyclo [4.3.0] nonène-5 et le diaza-1,5 bicyclo [5.4.0] undecène-5. Une fois la réaction terminée, on isole le produit de dépolymérisation formé, par exemple par précipitation par addition de chlorure de sodium, puis de méthanol.

La réaction entre l'ester et la base peut également être effectuée, en particulier dans le cas où les groupes acides non estérifiés de l'ester sont sous forme de sel d'ammonium quaternaire à longue chaîne, dans un solvant organique inerte dudit ester, tel que par exemple le dichlorométhane, le diméthylformamide, le formamide ou le tétrahydrofuranne, à une température de préférence de 20°C à 80°C. Comme bases utilisables on peut citer les bases solubles dans le solvant utilisé et en particulier le diaza-1,5 bicyclo [4.3.0] nonène-5, la quinuclidine et le diaza-1,5 bicyclo [5.4.0] undecène-5. Une fois la réaction terminée, on isole le produit de dépolymérisation formé, dans lequel les groupes acide carboxylique sont encore estérifiés, à l'état de sel alcalin, et l'hydrolyse par une solution aqueuse d'hydroxyde alcalin, en particulier d'hydroxyde de sodium, au moins 1 N, à basse température (0°C à +5°C). Le produit final est séparé, par exemple par précipitation par addition de chlorure de sodium, puis de méthanol.

Les esters d'héparine utilisés comme produits de départ pour la préparation des mélanges de polysaccharides selon l'invention peuvent être des esters non sélectifs ou sélectifs. Par esters non sélectifs on entend dans le cadre de la présente invention des esters d'héparine dans lesquels sont estérifiés indifféremment les groupes carboxyliques des chaînons acide D-glucuronique, acide L-iduronique non sulfaté et acide L-iduronique sulfaté. Par esters sélectifs on entend dans le cadre de la présente invention des esters d'héparine dans lesquels sont estérifiés, partiellement ou totalement, soit uniquement les groupes carboxyliques des chaînons acide D-glucuronique, soit uniquement les groupes carboxyliques des chaînons acide D-glucuronique et acide L-iduronique non sulfaté, soit encore uniquement les groupes carboxyliques des chaînons acide L-iduronique non sulfaté et acide L-iduronique sulfaté, soit enfin uniquement les groupes carboxyliques des chaînons acide L-iduronique sulfaté.

Comme esters d'héparine utilisables comme produit de départ dans les procédés selon l'invention, on peut citer en particulier les esters d'héparine décrits dans le brevet français 2 150 724 et dans le brevet anglais 1 501 095, ainsi que les esters méthylique, éthylique, éthoxycarbonylméthylique, cyanométhylique, benzylique et benzylique substitué (en particulier chloro-4 benzylique, nitro-4 benzylique) d'héparine. On emploie de préférence comme produit de départ les esters benzylique ou benzylique substitué d'héparine. Les esters d'héparine utilisés comme produits de départ dans les procédés selon l'invention peuvent être issus d'héparine de n'importe quelle origine (héparine de poumon de boeuf, héparine de muqueuse de porc, héparine d'intestins de boeuf, etc...).

Les esters méthylique, éthylique, éthoxycarbonylméthylique, cyanométhylique, benzylique et benzylique substitué d'héparine non sélectifs peuvent être obtenus par exemple par action d'un sel neutre d'ammonium quaternaire ou d'amine de l'héparine avec un dérivé halogéné de formule Hal—$CH_2$—R (II) dans laquelle Hal désigne un atome de chlore, de brome ou d'iode det R désigne un atome d'hydrogène ou un groupe méthyle, éthoxycarbonyl, cyano, phényle ou phényle substitué. Cette réaction est effectuée en solution ou en suspension dans un solvant inerte tel que le diméthylformamide, le clorure de méthylène, le diméthylsulfoxyde, le tétrahydrofuranne ou l'acétone, à une température comprise entre −20°C et +60°C.

Les esters méthylique, éthylique, éthoxycarbonylméthylique, cyanométhylique, benzylique et benzylique substitué d'héparine dans lesquels sont esterifiés, partiellement ou totalement, soit uniquement les groupes carboxyliques des chaînons acide D-glucuronique, soit uniquement les groupes carboxyliques des chaînons acide D-glucuronique et acide L-iduronique non sulfaté, sont obtenus par réaction d'un dérivé halogéné de formule (II) tel que défini précédemment avec un sel acide d'ammonium quaternaire de l'héparine dans lequel sont salifiés, outre les groupes sulfate, soit uniquement les groupes carboxyliques des chaînons acide D-glucuronique, soit uniquement les groupes carboxyliques des chaînons acide D-glucuronique et acide L-iduronique non sulfaté, les autres groupes carboxyliques étant sous forme acide. Cette réaction est effectuée dans les mêmes conditions que la réaction du dérivé halogéné de formule (II) avec un sel neutre d'ammonium quaternaire de l'héparine.

Les sels acides d'ammonium quaternaire de l'héparine, dans lesquels sont salifiés, outre les groupes sulfate, uniquement les groupes carboxyliques des chaînons acide D-glucuronique, sont obtenus par action d'un sel d'ammonium quaternaire sur l'héparine, en milieu aqueux, à un pH compris entre 3 et 4.

Les sels acides d'ammonium quaternaire de l'héparine, dans lesquels sont salifiés, outre les groupes sulfate, uniquement les groupes carboxyliques ques chaînons acides D-glucuronique et L-iduronique non sulfaté, sont obtenus par action d'un sel d'ammonium quaternaire sur l'héparine, en milieu aqueux, à un pH suffisamment bas pour former le sel d'ammonium quaternaire de l'héparine dans lequel seuls sont salifiés les groupes sulfate (en pratique pH 2 à 2,5), puis neutralisation sélective des groupes carboxyliques des chaînons acide D-glucuronique et acide L-iduronique non sulfaté du produit ainsi obtenu par addition d'une quantité calculée d'hydroxyde d'ammonium quaternaire en milieu diméthylformamide. Cette quantité est calculée en se basant sur la courbe de neutralisation d'une prise de poids déterminé dudit produit en milieu diméthylformamide.

Les esters méthylique, éthylique, éthoxycarbonylméthylique, cyanométhylique, benzylique et benzylique substitué d'héparine dans lesquels sont estérifiés, partiellement ou totalement, soit uniquement les groupes carboxyliques des chaînons acide L-iduronique sulfaté soit uniquement les groupes carboxyliques des chaînons acide L-iduronique non sulfaté et acide L-iduronique sulfaté sont préparés par action d'un alcool de formule HO—$CH_2$—R (III), dans laquelle R est un atome d'hydrogène, ou un groupe méthyle, éthoxycarbonyl, cyano, phényle ou phényle substitué, sur l'héparine, en milieu aqueux, en présence d'un agent de condensation du type carbodiimide soluble dans l'eau tel que par exemple l'éthyl-1 (diméthylamino-3 propyl)-3 carbodiimide, le pH du milieu étant ajusté à une valeur comprise dans l'intervalle 3,5—4,5 dans le premier cas et à une valeur comprise dans l'intervalle 2—3 dans le deuxième cas. Comme alcool de formule (III) utilisable on peut citer en particulier le méthanol et l'éthanol, auquel cas on obtient respectivement des esters d'héparine sélectifs méthylique et éthylique.

Certains des esters d'héparine cités précédemment, en particulier les esters dits sélectifs, sont des produits nouveaux, qui sont décrits et revendiqués, ainsi que leurs procédés de préparation, dans la demande de brevet européen 81 400 727.4 déposée le 8 mai 1981 au nom de la demanderesse, revendiquant le bénéfice de la priorité de la demande française 80/10792 du 14 mai 1980.

Les procédés selon l'invention utilisant l'action d'une base minérale ou organique sur un ester d'héparine, qui sont des procédés par $\beta$-élimination, permettent de réaliser une dépolymérisation partielle et contrôlée de l'héparine sans altérer sa structure générale.

Les mélanges de polysaccharides sulfatés selon l'invention possèdent des activités anticoagulante et antithrombotique et une activité hypolipémiante. Pour les mélanges de poids moléculaire moyen suffisamment faible (en pratique inférieur ou égal à 7000 daltons), l'activité antithrombotique est supérieure à l'activité anticoagulante. Les mélanges selon l'invention sont peu toxiques. A titre d'exemple, le produit de l'exemple 9 est atoxique à la dose de 300 mg/kg chez le rat et la souris, lorsqu'il est administré par la voie intra-veineuse, et présente une toxicité identique à celle de l'héparine, lorsqu'il est administré par la voie souscutanée.

Les mélanges de polysaccharides sulfatés selon l'invention dans lesquels les groupes acides des polysaccharides sont sous forme de sels pharmaceutiquement acceptables, en particulier sous forme de sel de sodium, de calcium ou de magnésium, sont utilisables, en tant qu'agents anticoagulants et antithrombotiques, pour la prévention et le traitement des thromboses. Ils sont également utilisables pour le traitement de l'hyperlipémie. Ils peuvent être avantageusement substitués à l'héparine pour de telles applications. En effet, administrés par voie sous-cutanée, ils présentent une plus longue durée d'action que l'héparine, ce qui permet de diminuer la fréquence des injections. D'autre part, ils provoquent moins d'effets secondaires (effets hémorragiques) que l'héparine.

Ils peuvent être administrés, en mélange avec un véhicule pharmaceutiquement acceptable, par voie intra-veineuse, par voie sous-cutanée, par voie pulmonaire (inhalation), par voie rectale, et, pour les mélanges de poids moléculaire moyen suffisamment bas (mélanges de la catégorie III en particulier), par voie orale. Les doses administrées dépendent du mode d'administration et de l'effet recherché (effet anti-thrombotique ou hypolipémiant).

Les exemples suivants illustrent l'invention sans la limiter. Le sel neutre de benzéthonium d'héparine ou héparinate de benzéthonium utilisé comme produit de départ dans les exemples 1 à 3, 10, 12 à 15 est issu d'une héparine d'intestin de porc ayant les caractéristiques suivantes:

| | |
|---|---|
| — poids moléculaire moyen en poids: | 16 000 daltons |
| — pouvoir rotatoire spécifique en solution aqueuse à 20°C: | $[\alpha]_D^{20} = +41°$ |
| — activité anticoagulante Codex: | 157 u.i./mg |

Le sel neutre de benzéthonium d'héparine ou héparinate de benzéthonium utilisé comme produit de départ dans les exemples 4 à 9 et 11 est issu d'une héparine d'intestin de boeuf ayant les caractéristiques suivantes:

| | |
|---|---|
| — poids moléculaire moyen en poids: $[\alpha]_D^{20} = +37°$ | 11 400 daltons |
| — activité anticoagulante Codex: | 128 u.i./mg |

Le sel neutre de benzéthonium d'héparine ou héparinate de benzéthonium utilisé comme produit de départ dans l'exemple 16 est issu d'une héparine de muqueuse de porc ayant un poids moléculaire moyen en poids de 16 000 daltons, un pouvoir rotatoire spécifique en solution aqueuse à 20°C de + 44° et une activité anticoagulante Codex de 180 u.i./mg.

Le sel de sodium d'héparine utilisé comme produit de départ dans les exemples 17 à 19 correspond à l'héparine de muqueuse de porc ci-dessus.

## Exemple 1

A une solution de 30 g d'héparinate de benzéthonium dans 600 ml de diméthylformamide on ajoute 30 g de chlorure de (chloro-4)benzyle. Après dissolution, on laisse en contact 60 heures à la température ambiante (environ 20°C) puis ajoute 600 ml d'une solution à 10% d'acétate de sodium dans le méthanol. Le précipité formé est séparé par filtration, lavé au méthanol et séché sous vide. On obtient ainsi 10,75 g d'ester chloro-4 benzylique d'heparine, sous forme de sel de sodium.

On met en contact, sous agitation, l'ester obtenu avec 269 ml d'une solution aqueuse 0,4 N d'hydroxyde de sodium à 25°C. Au bout de 2 heures on neutralise par addition d'une solution aqueuse 0,4 N

d'acide chlorhydrique et précipite par addition de deux volumes (c'est-à-dire le double du volume de la phase aqueuse) de méthanol. On isole par fitration 8,46 g d'héparine dépolymérisée, sous forme de sel de sodium.

## Exemple 2

A une solution de 30 g d'héparinate de benzéthonium dans 600 ml de diméthylformamide on ajoute 30 g de chlorure de benzyle. Après dissolution, on laisse en contact 60 heures à la température ambiante, puis précipite par addition de 600 ml d'une solution à 10% d'acétate de sodium dans le méthanol. On isole le précipité par filtration, le lave au méthanol et le sèche sous vide. On obtient ainsi 11,4 g d'ester benzylique d'héparine sous forme de sel de sodium.

On met en contact pendant deux heures, sous agitation, 3 g d'ester ci-dessus avec 75 ml d'une solution aqueuse 0,4 N d'hydroxyde de sodium à 20°C—25°C. Puis on neutralise par addition d'une solution aqueuse 0,4 N d'acide chlorhydrique et précipite par addition de 2 volumes de méthanol. On isole ainsi 2,23 g d'héparine dépolymérisée, sous forme de sel de sodium.

## Exemple 3

A une solution de 10 g d'héparinate de benzéthonium dans 250 ml de dichlorométhane on ajoute 10 g de chlorure de benzyle.

Après dissolution, on abandonne 24 heures à la température ambiante, puis évapore le solvant sous vide. Le résidu est dissous dans 150 ml de diméthylformamide et on précipite par addition de 150 ml d'une solution d'acétate de sodium à 10% dans le méthanol. On sépare par filtration 3,67 g d'ester benzylique d'héparine, sous forme de sel de sodium.

On traite sous agitation, pendant 2 heures, 2 g de l'ester ci-dessus avec 50 ml d'une solution aqueuse 0,1 N d'hydroxyde de sodium à 60°C. Après refroidissement, on neutralise par addition d'une solution aqueuse 0,1 N d'acide chlorhydrique, puis précipite par addition de 2 volumes de méthanol. On obtient ainsi 1,54 g d'héparine dépolymérisée, sous forme de sel de sodium.

## Exemple 4

A une solution de 5 g d'héparinate de benzéthonium dans 125 ml de dichlorométhane on ajoute 5 g de chloracétate d'éthyle et, après dissolution, on laisse en contact pendant 3 jours à la température ambiante. On évapore le solvant sous vide, reprend le résidu par 75 ml de diméthylformamide et précipite par addition de 75 ml d'une solution à 10% d'acétate de sodium dans le méthanol. Le précipité, séparé par filtration, est lave au méthanol, puis séché sous vide. On obtient ainsi 1,72 g d'ester carbéthoxyméthylique d'héparine, sous forme de sel de sodium.

On traite 1,7 g de l'ester ci-dessus par 43 ml d'une solution aqueuse 0,1 N d'hydroxyde de sodium à 60°C, sous agitation, pendant deux heures. Après refroidissement, on neutralise par addition d'une solution aqueuse 0,1 N d'acide chlorhydrique et précipite par addition de deux volumes de méthanol. On isole par filtration 1,33 g d'héparine dépolymérisée, sous forme de sel de sodium.

## Exemple 5

A une solution de 10 g d'héparinate de benzéthonium dans 250 ml de dichlorométhane on ajoute 10 g de chlorure de (chloro-4)benzyle, que l'on dissout par agitation. On abandonne la solution pendant 24 heures à température ambiante puis évapore le solvant sous vide. Le résidu est repris par 150 ml de diméthylformamide et on précipite par addition de 150 ml de solution à 10% d'acétate de sodium dans le méthanol. Après filtration, lavage du précipité au méthanol et séchage sous vide, on isole 3,84 g d'ester chloro-4 benzylique d'héparine, sous forme de sel de sodium.

On traite 2 g de l'ester ci-dessus par 50 ml d'une solution aqueuse 0,1 N d'hydroxyde de sodium à 60°C, sous agitation, pendant deux heures. Après refroidissement et neutralisation par une solution aqueuse 0,1 N d'acide chlorhydrique, on précipite par addition de deux volumes de méthanol. On isole le précipité par filtration, le lave au méthanol et le sèche sous vide. On obtient ainsi 1,38 g d'héparine dépolymérisée, sous forme de sel de sodium.

## Exemple 6

A une solution de 5 g d'héparinate de benzéthonium dans 125 ml de dichlorométhane on ajoute 5 g de chlorure de (nitro-4)benzyle, que l'on dissout par agitation. La solution est ensuite abandonnée pendant

3 jours à la température ambiante, puis on évapore le solvant sous vide et dissout le résidu dans 75 ml de diméthylformamide. On précipite l'ester formé par addition de 75 ml de solution à 10% d'acétate de sodium dans le méthanol. Le précipité est recueilli par filtration, lavé au méthanol et séché sous vide. On obtient ainsi 1,89 g d'ester nitro-4 benzylique d'héparine, sous forme de sel de sodium.

On traite 1,85 g de l'ester ci-dessus par 46 ml d'une solution aqueuse 0,1 N d'hydroxyde de sodium à 60°C, pendant deux heures, sous agitation. Après refroidissement, on neutralise par addition d'une solution aqueuse 0,1 N d'acide chlorhydrique, puis précipite par addition de deux volumes de méthanol. Le précipité est isolé par filtration, lavé au méthanol et séché sous vide. On obtient ainsi 1,13 g d'héparine dépolymerisée, sous forme de sel de sodium.

## Exemple 7

A une solution de 30 g d'héparinate de benzéthonium dans 500 ml de dichlorométhane on ajoute 30 g de chlorure de benzyle, que l'on dissout par agitation. La solution est ensuite abandonnée à température ambiante pendant 24 heures, puis on évapore le solvant sous vide et reprend le résidu par 400 ml d'éther. L'insoluble est séparé par filtration. On obtient ainsi 30 g d'ester benzylique d'héparine, sous forme de sel de benzéthonium. Cet ester est dissous dans 200 ml de dichlorométhane contenant 8 ml de diaza-1,5 bicyclo [4.3.0.] nonène-5. On porte à reflux pendant 3h30, puis évapore le solvant sous vide. Le résidu est dissous dans 450 ml de diméthylformamide et on ajoute un égal volume d'une solution à 10% d'acétate de sodium dans le méthanol. Le précipité est recueilli par filtration et lavé au méthanol. Puis on le traite à 0°C pendant une heure par une solution aqueuse 1 N d'hydroxyde de sodium. Après neutralisation, on précipite par addition de deux volumes de méthanol. Le précipité est isolé par filtration, lavé au méthanol et séché sous vide.

On obtient 6,6 g d'héparine dépolymérisée, sous forme de sel de sodium.

## Exemple 8

A une solution de 10 g d'héparinate de benzéthonium dans 250 ml de dichlorométhane on ajoute 10 g de chlorure de (chloro-4)benzyle, que l'on dissout par agitation. La solution est abandonnée à température ambiante pendant 24 heures, puis on évapore le solvant sous vide. On reprend le résidu par 200 ml d'éther et isole par filtration le précipité formé. On obtient ainsi 10 g d'ester chloro-4 benzylique d'héparine, sous forme de sel de benzéthonium.

On dissout 5 g de ce produit dans 100 ml de dichlorométhane contenant 1,5 ml de diaza-1,5 bicyclo [4.3.0] nonène-5 et porte à reflux pendant 4 heures. Puis on évapore le solvant sous vide, reprend le résidu par 30 ml de diméthylformamide et ajoute 100 ml d'une solution à 10% d'acétate de sodium dans le méthanol. Le précipité formé est isolé par filtration, lavé au méthanol, puis traité par 24 ml d'une solution aqueuse 1 N d'hydroxyde de sodium pendant une heure à 0°C. On neutralise par addition d'une solution aqueuse 1 N d'acide chlorhydrique et précipite par addition de deux volumes de méthanol. On sépare le précipité par filtration. Après lavage au méthanol et séchage sous vide, on obtient 1 g d'héparine dépolymérisée, sous forme de sel de sodium.

## Exemple 9

A une solution de 30 g d'héparinate de benzéthonium dans 600 ml de diméthylformamide on ajoute 30 g de chlorure de benzyle. Après dissolution, on laisse en contact pendant 60 heures à la température ambiante, puis on précipite l'ester formé par addition de 1200 ml d'une solution à 10% d'acétate de sodium dans le méthanol. Le précipité est isolé par filtration, lavé au méthanol et séché sous vide. On obtient ainsi 11,4 g d'ester benzylique d'héparine, sous forme de sel de sodium.

On traite 10 g de l'ester ci-dessus par 250 ml d'une solution aqueuse 0,1 N d'hydroxyde de sodium à 60°C, pendant deux heures sous agitation. Après refroidissement, on neutralise par une solution aqueuse 0,1 N d'acide chlorhydrique et précipite par addition de deux volumes de méthanol. Le précipité est filtré, lavé au méthanol et séché sous vide. On obtient ainsi 6,65 g d'héparine dépolymérisée, sous forme de sel de sodium.

## Exemple 10

A une solution de 120 g d'héparinate de benzéthonium dans 2,4 l de diméthylformamide on ajoute 120 g de chlorure de (chloro-4)benzyle, que l'on dissout par agitation. La solution est ensuite abandonnée à température ambiante pendant 60 heures, puis on ajoute 2,4 l d'une solution à 10% d'acétate de sodium dans du méthanol. Le précipité formé est séparé par filtration, lavé au méthanol et séché sous vide. On obtient ainsi 46 g d'ester chloro-4 benzylique d'héparine, sous forme de sel de sodium.

On traite 20 g de l'ester ci-dessus par 500 ml d'une solution aqueuse 0,1 N d'hydroxyde de sodium à 60°C, pendant deux heures, sous agitation. Après refroidissement et neutralisation, on précipite par addition de deux volumes de méthanol. On isole par filtration 11,7 g d'héparine dépolymérisée, sous forme de sel de sodium.

## Exemple 11

A une solution de 30 g d'héparinate de benzéthonium dans 750 ml de dichlorométhane on ajoute 30 g d'iodure de méthyle, que l'on dissout par agitation. On abandonne la solution pendant 48 heures à température ambiante, puis évapore le solvant sous vide. Le résidu est repris par 450 ml de diméthylformamide et on précipite par addition de 450 ml de solution à 10% d'acétate de sodium dans le méthanol. Après filtration, le précipité est lavé au méthanol et séché sous vide. On isole ainsi 10,5 g d'ester méthylique d'héparine, sous forme de sel de sodium.

On traite 2 g de l'ester ci-dessus par 50 ml d'une solution aqueuse 0,1 N d'hydroxyde de sodium à 60°C, sous agitation, pendant deux heures. Après refroidissement, on amène le pH de la solution à 4,5 environ par agitation avec une résine échangeuse d'ions carboxylique sous forme $H^+$. La résine est ensuite séparée par filtration et lavée à l'eau. Les phases aqueuses rassemblées sont neutralisées par addition d'une solution aqueuse diluée d'hydroxyde de sodium, puis on lyophilise. On obtient ainsi 2 g d'héparine dépolymérisée, sous forme de sel de sodium.

## Exemple 12

On traite 3 g de l'ester chloro-4 benzylique d'héparine obtenu à l'exemple 10 par 120 ml d'une solution aqueuse à 10% de carbonate disodique à 60°C, pendant deux heures, sous agitation. Après refroidissement, on neutralise par une solution aqueuse 0,4 N d'acide chlorhydrique et précipite par addition de deux volumes de méthanol. On isole par filtration 1,57 g d'héparine dépolymérisée, sous forme de sel de sodium.

## Exemple 13

A une solution de 30 g d'héparinate de benzéthonium dans 600 ml de diméthylformamide on ajoute 30 g de chloracétate d'éthyle. Après dissolution, on laisse en contact 60 heures à la température ambiante, puis ajoute 600 ml d'une solution à 10 % d'acétate de sodium dans du méthanol. Le précipité formé est séparé par filtration, lavé au méthanol et séché sous vide. On obtient ainsi 10,78 g d'ester carbéthoxyméthylique d'héparine, sous forme de sel de sodium.

On met en contact, sous agitation, 3 g de l'ester ci-dessus avec 100 ml d'une solution aqueuse à 3 % de triéthylamine à la température de 60°C. Au bout de 5 heures, on neutralise par addition d'une solution aqueuse d'acide chlorhydrique, puis précipite par addition de 2 volumes de méthanol. On isole par filtration 2,5 g d'héparine dépolymérisée sous forme de sel de sodium.

## Exemple 14

A une solution de 5 g d'héparinate de brenzéthonium dans 125 ml de dichlorométhane on ajoute 5 g de chloracétonitrile, que l'on dissout par agitation. On abandonne la solution pendant 48 heures à température ambiante, puis on évapore le solvant sous vide. Le résidu est dissous dans 75 ml de diméthylformamide et on précipite par addition de 75 ml d'une solution à 10% d'acétate de sodium dans le méthanol. Le Précipité est séparé par filtration, lavé au méthanol et séché sous vide. On obtient ainsi 1,63 g d'ester cyanométhylique d'héparine, sous forme de sel de sodium.

On traite l'ester obtenu par 40 ml d'une solution aqueuse 0,1 N d'hydroxyde de sodium à 60°C, sous agitation, pendant deux heures. Après refroidissement, on neutralise par addition d'une solution aqueuse 0,1 N d'acide chlorhydrique, puis précipite par addition de deux volumes de méthanol. On isole par filtration 1,33 g d'héparine dépolymérisée, sous forme de sel de sodium.

## Exemple 15

On dissout sous agitation 3 g de l'ester chloro-4 benzylique d'héparine obtenu à l'exemple 10 dans 120 ml d'une solution aqueuse à 10% de carbonate disodique. Après deux heures d'agitation à une température de 20°C à 25°C, on amène le pH de la solution à 6 par addition d'une solution aqueuse N d'acide chlorhydriques, puis ajoute un volume de méthanol égal à deux fois le volume de la solution aqueuse. On isole par filtration le précipité formé et obtient ainsi 2,1 g d'ester chloro-4 benzylique d'héparine.

On traite sous agitation, pendant deux heures, 2 g de l'ester ci-dessus par 50 ml d'une solution aqueuse 0,1 N d'hydroxyde de sodium à 60°C. Après refroidissement, on neutralise par addition d'une solution aqueuse 0,1 N d'acide chlorhydrique, puis précipite par addition de deux volumes de méthanol. On obtient ainsi 1,4 g d'héparine dépolymérisée, sous forme de sel de sodium.

Dans les exemples 1 à 10 et 12 à 15 précédents, avant de précipiter le produit formé par addition des deux volumes de méthanol, on a ajusté la concentration en NaCl de la phase aqueuse à 10% par addition de chlorure de sodium.

## Exemple 16

A une solution de 10 g d'héparinate de benzéthonium dans 250 ml de dichlorométhane, on ajoute 10 g de chlorure de (chloro-4) benzyle que l'on dissout par agitation. La solution est abandonnée à température ambiante pendant 24 heures, puis on précipite l'ester formé par addition d'une solution à 10% d'acétate de sodium dans du méthanol. Le précipité est filtré, lavé au méthanol. On obtient ainsi 3,72 g d'ester chloro-4 benzylique d'héparine, sous forme de sel de sodium.

On traite à 60°C, pendant 5 heures, une solution de 0,500 g de l'ester précédent dans 10 ml de formamide par 0,5 ml de diaza-1,5 bicyclo [4,3,0] nonène-5. Après refroidissement, on précipite par addition de 70 ml d'acétone. On recueille ainsi 0,364 g d'un produit qui est traité à 0°C, pendant 2 heures, par 6 ml d'une solution aqueuse N d'hydroxyde de sodium. La phase aqueuse est neutralisée par addition d'une solution aqueuse N d'acide chlorhydrique et sa concentration en NaCl est ajustée à 10% par addition de chlorure de sodium. On précipite par addition de deux volumes de méthanol. On isole ainsi 0,263 g d'héparine dépolymérisée, sous forme de sel de sodium.

## Exemple 17

A une solution de 10 g d'héparine (sel de sodium) dans 40 ml d'eau on ajoute 2,5 ml d'acide acétique puis, lentement et sous agitation, 150 ml d'une solution aqueuse à 10% de chlorure de benzéthonium. Le précipité formé est recueilli par centrifugation, lavé à l'eau et séché sous vide. On obtient 19,67 g d'héparinate acide de benzéthonium.

On traite 11 g du produit par 11 g de chlorure de (chloro-4) benzyle dissous dans 110 ml de diméthylformamide, pendant 48 heures, à la température ambiante. On ajoute 220 ml d'une solution à 10% d'acétate de sodium dans le méthanol. Le précipité formé est filtré et lavé au méthanol. On obtient ainsi 4,70 g d'ester (chloro-4) benzylique d'héparine, sous forme de sel de sodium.

4 g de l'ester ci-dessus sont dissous dans 20 ml d'eau et on ajoute lentement, sous agitation, 40 ml d'une solution aqueuse à 20% da chlorure de benzéthonium. Le précipité formé est recueilli par centrifugation, lavé à l'eau et séché sous vide. On obtient ainsi l'ester (chloro-4) benzylique d'héparine, sous forme de sel de benzéthonium.

1 g de l'ester ci-dessus sous forme de sel de benzéthonium est dissous dans 20 ml de diméthylformamide et est traité par 1 ml de diaza-1,5 bicyclo [4,3,0] nonène-5 à 60°C, pendant 5 heures. Après refroidissement, on précipite par addition de 50 ml d'une solution à 10% d'acétate de sodium dans le méthanol. On obtient ainsi 0,346 g d'un produit qui est traité à 0°C, pendant deux heures, par 5,8 ml d'une solution aqueuse N d'hydroxyde de sodium. La phase aqueuse est neutralisée par addition d'une solution aqueuse N d'acide chlorhydrique et sa concentration en NaCl est ajustée à 10% par addition de chlorure de sodium. On précipite par addition de deux volumes de méthanol. Après filtration et lavage au méthanol du précipité, on obtient 0,253 g d'héparine dépolymérisée sous forme de sel de sodium.

## Exemple 18

A une solution de 10 g d'héparine (sel de sodium) dans 40 ml d'eau on ajoute 2,5 ml d'acide formique puis, lentement et sous agitation, 150 ml d'une solution aqueuse à 10% de chlorure de benzéthonium. On recueille le précipité par centrifugation, le lave à l'eau et le sèche sous vide. On obtient ainsi 20,5 g d'héparinate acide de benzéthonium. On dissout 2,95 g du produit précédent dans 60 ml de diméthylformamide, puis on ajoute 5,9 ml d'une solution 0,1 N d'hydroxyde de tétrabutylammonium dans un mélange n-propanol/méthanol.

Après addition de 2,95 g de chlorure de (chloro-4) benzyle on abandonne cinq jours à température ambiante. On ajoute 74 ml d'une solution à 10% d'acétate de sodium dans le méthanol. On isole par filtration 1,32 g d'ester (chloro-4) benzylique d'héparine, sous forme de sel de sodium.

L'ester ci-dessus est dissous dans 6,4 ml d'eau et on ajoute lentement, sous agitation, 12,8 ml d'une solution aqueuse à 20% de chlorure de benzéthonium. Le précipité formé est recueilli par centrifugation, lavé à l'eau et séché sous vide. On obtient ainsi l'ester chloro-4 benzylique d'héparine, sous forme de sel de benzéthonium.

1 g de l'ester ci-dessus sous forme de sel de benzéthonium est dissous dans 20 ml de dichlorométhane et on ajoute 1 ml de diaza-1,5 bicyclo [4,3,0] nonène-5. On chauffe au reflux pendant 5 heures, puis évapore le solvant sous vide, reprend le résidu par 15 ml de diméthylformamide et ajoute 20 ml d'une solution à 10% d'acétate de sodium dans le méthanol. Le précipité formé est isolé par filtration et lavé au méthanol.

On obtient sinsi 0,405 g d'un produit qui est traité à 0°C, pendant deux heures, par 6 ml d'une solution aqueuse N d'hydroxyde de sodium. La phase aqueuse est netralisée par addition d'une solution aqueuse N d'acide chlorhydrique et sa concentration en NaCl est ajustée à 10% par addition de chlorure de sodium. On précipite par addition de deux volumes de méthanol. Après filtration et lavage au méthanol du précipité, on obtient 0,355 g d'héparine dépolymérisée sous forme de sel de sodium.

Exemple 19

On dissout 0,600 g d'héparine (sel de sodium) dans 7 ml d'eau et ajuste le pH de la solution à 3,5 par addition d'une solution aqueuse N d'acide chlorhydrique. Puis on ajoute 0,300 g d'éthyl-1 (diméthylamino-3-propyl)-3 carbodiimide et, après dissolution, abandonne la solution pendant une heure à la température ambiante. On ajoute 2,5 ml d'une solution aqueuse de chlorure de sodium à 280 g/l, puis 15 ml de méthanol. Le précipité formé est isolé par filtration, lavé au méthanol et séché sous vide. On obtient ainsi 0,527 g d'ester méthylique d'héparine, sous forme de sel de sodium, dont le taux d'estérification des groupes carboxyliques est 50%.

On dissout 0,300 g de l'ester précédent dans 7,5 ml d'une solution aqueuse 0,1 N d'hydroxyde de sodium et chauffe à 60°C pendant deux heures. Après refroidissement on neutralise par addition d'une solution aqueuse 0,1 N d'acide chlorhydrique, ajuste la concentration en NaCl du milieu à 10% par addition de chlorure de sodium et précipite par addition de deux volumes de méthanol. On obtient ainsi 0,200 g d'héparine dépolymérisée, sous forme de sel de sodium.

Les tableaux B et C suivants donnent les caractéristiques des produits (héparines dépolymérisées sous forme de sel de sodium) préparés dans les exemples 1 à 19. Les pourcentages de soufre, d'azote et d'acides uroniques, les pouvoirs rotatoires spécifiques en solution aqueuse à 20°C, les poids moléculaires moyens en poids et les activités indiqués dans ce tableau ont été déterminés par les méthodes signalées précédemment. Dans la colonne dispersion de poids moléculaires figurent les valeurs extrêmes approximatives des poids moléculaires des polysaccharides constitutifs des mélanges, telles que déterminées par chromatographie par perméation de gel sur gel de polyacrylamide-agarose. Dans la colonne viscosité figurent les viscosités à 25°C des solutions aqueuses à 10% des produits. Dans la colonne absorption UV figurent les absorptions, sous une épaisseur de 1 cm, de solutions à 1 % des produits dans HCl 0,01 N, l'absorption étant mesurée au maximum d'absorption situé dans la zone 220–232 nm.

Tableau C

| Exemple | Poids moléculaire moyen (en daltons) | Absorption UV | Activité anticoagulante anti Xa in vitro | APTT in vithro | Codex in vitro |
|---|---|---|---|---|---|
| 16 | 4200 | 6 | 140 | 63 | 60 |
| 17 | 5500 | 7,96 | 165 | 80 | 70 |
| 18 | 4400 | 6,3 | 110 | 60 | 40 |
| 19 | 5000 | 10,9 | 150 | 45 | 50 |

Tableau B

| Exemple | % acides ureniques | % S | % N | $[\alpha]_D^{20}$ | Poids moléculaire moyen (en daltons) | Dispersion de poids moléculaires |
|---|---|---|---|---|---|---|
| 1 | 23,0 | 11,5 | 2,1 | + 44° | 7 300 | 1 600–12 000 |
| 2 | 26,0 | 11,6 | 2,0 | + 44° | 6 500 | 1 800–13 000 |
| 3 | 25,4 | 12,0 | 2,2 | + 44° | 6 400 | 3 000–11 000 |
| 4 | 23,9 | 10,9 | 2,1 | + 35° | 5 500 | 2 200–13 000 |
| 5 | 22,8 | 12,2 | 2,2 | + 39° | 5 000 | 2 000–13 000 |
| 6 | 24,5 | 10,8 | 2,3 | + 33° | 4 200 | 1 000–10 000 |
| 7 | 25,2 | 11,3 | 2,0 | + 33° | 4 100 | 1 700–10 000 |
| 8 | 23,7 | 11,1 | 2,2 | + 35° | 4 500 | 1 000–10 000 |
| 9 | 26,0 | 11,8 | 2,2 | + 38° | 4 000 | 2 000–10 000 |
| 10 | 25,2 | 10,9 | 2,1 | + 41° | 3 800 | 1 000– 9 000 |
| 11 | 23,8 | 11,0 | 2,3 | + 27° | 2 800 | 1 000– 8 000 |
| 12 | 23,9 | 11,5 | 2,0 | + 40° | 4 000 | 2 000– 9 000 |
| 13 | 24,3 | 11,8 | 2,2 | + 45° | 9 000 | 3 000–20 000 |
| 14 | 25,6 | 12,0 | 2,1 | + 44° | 8 500 | 3 000–20 000 |
| 15 | 25,3 | 11,8 | 2,3 | + 45° | 4 000 | 2 000–20 000 |

Tableau B (suite)

| Exemple | Viscosité (en centipoises) | Absorption UV | Activité anti-coagulante Codex in vitro (u.i./mg) | Activité anti-Xa in vitro (u.i./mg) | Activité A.P.T.T. in vitro (u.i./mg) |
|---|---|---|---|---|---|
| 1 | 2,37 | 7,5 | 127 | 180 | 105 |
| 2 | 2,25 | 7,8 | 116 | 148 | 100 |
| 3 | 2,08 | 7,9 | 112 | 163 | 89 |
| 4 | 1,93 | 8,2 | 83 | 130 | 52 |
| 5 | 1,91 | 9,7 | 75 | 118 | 43 |
| 6 | 1,74 | 12,5 | 60 | 140 | 33 |
| 7 | 1,64 | 15,0 | 50 | 130 | 30 |
| 8 | 1,61 | 16,4 | 54 | 110 | 33 |
| 9 | 1,62 | 14,0 | 80 | 159 | 45 |
| 10 | 1,57 | 15,4 | 62 | 159 | 40 |
| 11 | 1,50 | 22,8 | 40 | 90 | 20 |
| 12 | 1,60 | 14,4 | 75 | 140 | 35 |
| 13 | 2,60 | 5,9 | 135 | 140 | 130 |
| 14 | 2,50 | 6,1 | 138 | 130 | 130 |
| 15 | 1,65 | 10,64 | 110 | 170 | 86 |

Exemple 20

Le produit de l'exemple 1 d'une part et une héparine commerciale d'autre part ont été administrés, à des époques différentes, par voie sous-cutanée, à cinq volontaires sains, à la dose de 5000 u. i. Codex. Sur des prélèvements sanguins effectués 1 heure, 3 heures, 5 heures et 7 heures après l'administration, on a mesuré l'activité anticoagulante plasmatique au moyen des tests anta-Xa et A. P. T. T. définis précédemment. Les résultats obtenus ont été exprimés en unités internationales par ml de plasma en se référant à une courbe d'étalonnage tracée à partir d'essais effectués sur un plasma témoin auquel on a ajouté des quantités connues d'héparine de référence (3éme étalon international).

Les résultats moyens obtenus sont rassemblés dans le tableau D suivant:

Tableau D

| Temps écoulé entra l'administration et le prélèvement | Activité anti-Xa | | Activité A. P. T. T. | |
|---|---|---|---|---|
| | Produit de l'exemple 1 | Héparine commerciale | Produit de l'exemple 1 | Héparine commerciale |
| 1 heure | 0,20 | 0,04 | 0,04 | 0,05 |
| 3 heures | 0,27 | 0,08 | 0,05 | 0,04 |
| 5 heures | 0,28 | 0,06 | 0,03 | 0,02 |
| 7 heures | 0,22 | 0,05 | 0,02 | 0,01 |

On voit que le produit de l'exemple 1 exerce un effet anti-Xa beaucoup plus intense que celui de l'héparine commerciale.

Dans le cadre de la présente demande, le dalton est défini comme étant égal au seizième de la masse d'un atome d'oxygène, soit $1{,}65 \times 10^{-24}$ gramme (of A. Manuila, L. Manuila, M. Nicole, H. Lambert, Dictionnaire Français de Médecine et de Biologie, Tome I, 1970, p. 744).

**Revendications**

1. Mélanges de polysaccharides sulfatés ayant la structure générale des polysaccharides constitutifs de l'héparine et dont les groupes acides sont sous forme libre ou salifiée, caractérisés en ce que les polysaccharides constitutifs desdits mélanges possèdent une double liaison éthylénique à l'une des extrémités de leur chaîne et en ce que les mélanges présentent, à l'état de sel de sodium, les caractéristiques suivantes:

| | |
|---|---|
| — pourcentage de soufre: | 9% à 13,5% |
| — pourcentage d'azote: | 1,8% à 2,5% |
| — pourcentage d'acides uroniques: | 20% à 30% |
| — poids moléculaire moyen en poide: | 2000 à 10000 daltons |
| — pouvoir rotatoire spécifique en solution aqueuse à 20°C: | $[\alpha]_D^{20} = + 25°$ à 55° |

2. Mélanges selon la revendication 1, caractérisés en ce que les polysaccharides constitutifs desdits mélanges répondent à la formule:

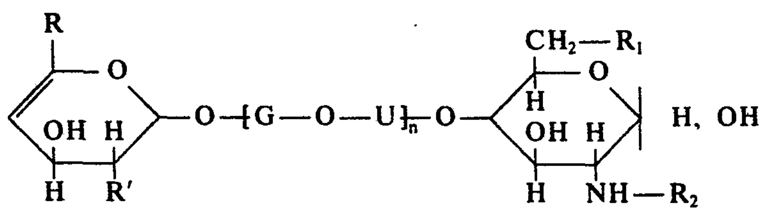

(I)

dans laquelle R désigne un atome d'hydrogène ou un groupe carboxylique, sous forme acide ou sous forme salifiée, R' désigne un groupe OH ou un groupe sulfate, sous forme acide ou sous forme salifiée, $R_1$ désigne un groupe OH ou un groupe sulfate, sous forme acide ou sous forme salifiée, $R_2$ désigne un groupe sulfonate, sous forme acide ou sous forme salifiée, ou un groupe acétyle, —O— désigne un pont oxygène, les chaînons G désignent les chaînons du type glucosamine figurant dans la structure de l'héparine, les chaînons U désignent les chaînons du type acide uronique (acide D-glucuronique, acide L-iduronique, acide L-iduronique sulfaté) figurant dans la structure de l'héparine, et n est un nombre entier de 3 à 20, les groupes acides des polysaccharides étant sous forme libre ou sous forme salifiée.

3. Mélanges selon l'une quelconque des revendications 1 et 2, caractérisés en ce qu'ils présentent, à l'état de sel de sodium, les caractéristiques suivantes:

| | |
|---|---|
| — poids moléculaire moyen en poids: | 8000 à 10000 daltons |
| — activité anticoagulante in vitro Codex: | 130 à 160 u.i./mg |
| — activité in vitro anti-Xa: | 130 à 180 u.i./mg |
| — avtivité in vitro A. P. T. T.: | 100 à 150 u.i./mg |

– rapport $\frac{\text{activité in vitro anti-Xa}}{\text{activité in vitro A.P.T.T.}}$ : 1 à 1,5

4. Mélanges selon l'une quelconque des revendications 1 et 2, caractérisés en ce qu'ils présentent, à l'état de sel de sodium, les caractéristiques suivantes:

| | |
|---|---|
| – poids moléculaire moyen en poids: | 3000 à 8000 daltons |
| – activité anticoagulante in vitro Codex: | 80 à 140 u.i./mg |
| – activité in vitro anti-Xa: | 120 à 250 u.i./mg |
| – avtivité in vitro A.P.T.T.: | 80 à 120 u.i./mg |
| – rapport $\frac{\text{activité in vitro anti-Xa}}{\text{activité in vitro A.P.T.T.}}$ : | 1,4 à 3 |

5. Mélanges selon l'une quelconque des revendications 1 et 2, caractérisés en ce qu'ils présentent, à l'état de sel de sodium, les caractéristiques suivantes:

| | |
|---|---|
| – poids moléculaire moyen en poids: | 2000 à 7000 daltons |
| – activité anticoagulante in vitro Codex: | 10 à 80 u.i./mg |
| – activité in vitro anti-Xa: | 80 à 250 u.i./mg |
| – avtivité in vitro A.P.T.T.: | 10 à 80 u.i./mg |
| – rapport $\frac{\text{activité in vitro anti-Xa}}{\text{activité in vitro A.P.T.T.}}$ : | 2 à 10 |

6. Mélange selon la revendication 5, caractérisé en ce qu'il présente, à l'état de sel de sodium, les caractéristique suivantes:

| | |
|---|---|
| – poids moléculaire moyen en poids: | 4200 daltons |
| – pouvoir rotatoire | $[\alpha]_D^{20} = +33°$ |
| – activité anticoagulante in vitro Codex: | 60 u.i./mg |
| – activité in vitro anti-Xa: | 140 u.i./mg |
| – activité in vitro A.P.T.T.: | 33 u.i./mg |

7. Mélange selon la revendication 5, caractérisé en ce qu'il présente, à l'état de sel de sodium, les caractéristique suivantes:

| | |
|---|---|
| – poids moléculaire moyen en poids: | 4100 daltons |
| – pouvoir rotatoire | $[\alpha]_D^{20} = +33°$ |
| – activité anticoagulante in vitro Codex: | 50 u.i./mg |
| – activité in vitro anti-Xa: | 130 u.i./mg |
| – activité in vitro A.P.T.T.: | 30 u.i./mg |

8. Mélange selon la revendication 5, caractérisé en ce qu'il présente, à l'état de sel de sodium, les caractéristiques suivantes:

| | |
|---|---|
| – poids moléculaire moyen en poids: | 4500 daltons |
| – pouvoir rotatoire | $[\alpha]_D^{20} = +35°$ |
| – activité anticoagulante in vitro Codex: | 54 u.i./mg |
| – activité in vitro anti-Xa: | 110 u.i./mg |
| – activité in vitro A.P.T.T.: | 33 u.i./mg |

9. Mélange selon la revendication 5, caractérisé en ce qu'il présente, à l'état de sel de sodium, les caractéristiques suivantes:

| | |
|---|---|
| – poids moléculaire moyen en poids: | 4000 daltons |
| – pouvoir rotatoire | $[\alpha]_D^{20} = +38°$ |
| – activité anticoagulante in vitro Codex: | 80 u.i./mg |
| – activité in vitro anti-Xa: | 159 u.i./mg |
| – activité in vitro A.P.T.T.: | 45 u.i./mg |

10. Mélange selon la revendication 5, caractérisé en ce qu'il présente, à l'état de sel de sodium, les caractéristiques suivantes:

| | |
|---|---|
| — poids moléculaire moyen en poids: | 3800 daltons |
| — pouvoir rotatoire | $[\alpha]_D^{20} = + 41°$ |
| — activité anticoagulante in vitro Codex: | 62 u.i./mg |
| — activité in vitro anti-Xa: | 159 u.i./mg |
| — activité in vitro A.P.T.T.: | 40 u.i./mg |

11. Mélange selon la revendication 5, caractérisé en ce qu'il présente, à l'état de sel de sodium, les caractéristiques suivantes:

| | |
|---|---|
| — poids moléculaire moyen en poids: | 2800 daltons |
| — pouvoir rotatoire | $[\alpha]_D^{20} = + 27°$ |
| — activité anticoagulante in vitro Codex: | 40 u.i./mg |
| — activité in vitro anti-Xa: | 90 u.i./mg |
| — activité in vitro A.P.T.T.: | 20 u.i./mg |

12. Mélange selon la revendication 5, caractérisé en ce qu'il présente, à l'état de sel de sodium, les caractéristiques suivantes:

| | |
|---|---|
| — poids moléculaire moyen en poids: | 4000 daltons |
| — pouvoir rotatoire | $[\alpha]_D^{20} = + 40°$ |
| — activité anticoagulante in vitro Codex: | 75 u.i./mg |
| — activité in vitro anti-Xa: | 140 u.i./mg |
| — activité in vitro A.P.T.T.: | 35 u.i./mg |

13. Mélange selon la revendication 4, caractérisé en ce qu'il présente, à l'état de sel de sodium, les caractéristiques suivantes:

| | |
|---|---|
| — poids moléculaire moyen en poids: | 4000 daltons |
| — pouvoir rotatoire | $[\alpha]_D^{20} = + 45°$ |
| — activité anticoagulante in vitro Codex: | 110 u.i./mg |
| — activité in vitro anti-Xa: | 170 u.i./mg |
| — activité in vitro A.P.T.T.: | 86 u.i./mg |

14. Procédé de préparation des mélanges selon la revendication 1, caractérisé en ce que l'on fait réagir, en milieu aqueux et à une température de 20°C à 80°C, un ester d'héparine, soluble dans l'eau, résultant de l'estérification partielle ou totale des groupes acides carboxyliques de l'héparine avec une base minérale ou organique soluble dans l'eau et isole le produit de dépolymérisation ainsi formé.

15. Procédé selon la revendication 14, caractérisé en ce que la concentration molaire de la base dans le milieu est 0,1 à 0,6.

16. Procédé de préparation des mélanges selon la revendication 1, caractérisé en ce que l'on fait réagir un ester d'héparine résultant de l'estérification partielle ou totale des groupes acide carboxylique de l'héparine avec une base, au sein d'un solvant organique inerte dudit ester, isole le produit de dépolymérisation ainsi formé à l'état de sel alcalin et l'hydrolyse par une solution aqueuse d'hydroxyde de sodium au moins 1 N à basse température.

17. Procédé selon la revendication 16, caractérisé en ce que la réaction de l'ester d'héparine avec la base est effectuée à une température de 20°C à 80°C.

18. Médicament utilisable en particulier pour la prévention et le traitement des thromboses et pour le traitement de l'hyperlipémie, caractérisé en ce qu'il contient comme ingrédient actif un mélange de polysaccharides sulfatés tel que défini dans chacune des revendications 1 à 13, les groupes acides desdits polysaccharides étant sous forme de sels pharmaceutiquement acceptables.

19. Médicament selon la revendication 18, caractérisé en ce que les groupes acides des polysaccharides sulfatés sont sous forme de sel de sodium, de sel de calcium ou de sel de magnésium.

## Patentansprüche

1. Gemische von sulfatierten Polysacchariden mit der allgemeinen Struktur der grundlegenden Polysaccharide des Heparins und deren saure Gruppen in freier oder in Salzform vorliegen, dadurch gekennzeichnet, daß die grundlegenden Polysaccharide dieser Gemische eine äthylenische Doppelbindung an einer der äußeren Enden ihrer Kette besitzen und daß die Gemische im Zustand des Natriumsalzes die folgenden Charakteristika aufweisen:

| | |
|---|---|
| — Protentsatz Schwefel: | 9 bis 13,5% |
| — Prozentsatz Stickstoff: | 1,8 bis 2,5% |
| — Prozentsatz Uronsäuren: | 20 bis 30% |

- mittleres Molekulargewicht in Gewicht: 2000 bis 10 000 Dalton
- spezifisches Drehvermögen in wäßriger Lösung bei 20°C: $[\alpha]_D^{20} = +25°$ bis $+55°$

2. Gemische gemäß Anspruch 1, dadurch gekennzeichnet, daß die grundlegenden Polysaccharide dieser Gemische der Formel (I) entsprechen:

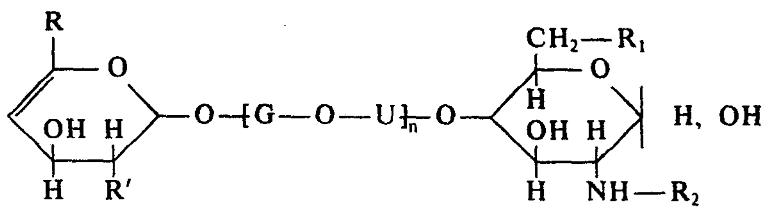

(I)

worin R ein Wasserstoffatom oder eine Carboxylgruppe in Säureform oder in Salzform bedeutet, R' eine Gruppe OH oder eine Sulfatgruppe, in Säureform oder in Salzform, bedeutet, $R_1$ eine Gruppe OH oder eine Sulfatgruppe in Säureform oder in Salzform bedeutet, $R_2$ eine Sulfonatgruppe in Säureform oder in Salzform oder eine Acetylgruppe bedeutet, $-O-$ eine Sauerstoffbrücke bedeutet, wobei die Kettenglieder G die Kettenglieder vom Glukosamintyp, welche in der Struktur des Heparins vorkommen, bedeuten und die Kettenglieder U die Kettenglieder vom Typ Uronsäure (D-Glukuronsäure, L-Iduronsäure, sulfatierte L-Iduronsäure), welche in der Struktur des Heparins vorkommen, bedeuten und n ist eine ganze Zahl von 3 bis 20, wobei die Säuregruppen der Polysaccharide in freier Form oder in Salzform vorhanden sind.

3. Gemische gemäß einem der Ansprüche 1 und 2, dadurch gekennzeichnet, daß sie im Zustand des Natriumsalzes die folgenden Charakteristika aufweisen:

- mittleres Molekulargewicht in Gewicht: 8000 bis 10 000 Dalton
- antikoagulierende Aktivität in vitro Codex: 130 bis 160 I.E./mg
- in vitro Aktivität anti-Xa: 130 bis 180 IE/mg
- in vitro Aktivität A.P.T.T.: 100 bis 150 IE/mg
- Verhältnis $\frac{\text{in vitro Aktivität anti-Xa}}{\text{in vitro Aktivität A.P.T.T.}}$: 1 bis 1,5

4. Gemische gemäß einem der Ansprüche 1 und 2, dadurch gekennzeichnet, daß sie im Zustand des Natriumsalzes die folgenden Charakteristika aufweisen:

- mittleres Molekulargewicht in Gewicht: 3000 bis 8000 Dalton
- antikoagulierende Aktivität in vitro Codex: 80 bis 140 I.E./mg
- in vitro Aktivität anti-Xa: 120 bis 250 IE/mg
- in vitro Aktivität A.P.T.T.: 80 bis 120 IE/mg
- Verhältnis $\frac{\text{in vitro Aktivität anti-Xa}}{\text{in vitro Aktivität A.P.T.T.}}$: 1,4 bis 3

5. Gemische gemäß einem der Ansprüche 1 und 2, dadurch gekennzeichnet, daß sie im Zustand des Natriumsalzes die folgenden Charakteristika aufweisen:

- mittleres Molekulargewicht in Gewicht: 2000 bis 7000 Dalton
- antikoagulierende Aktivität in vitro Codex: 10 bis 80 IE/mg
- in vitro Aktivität anti-Xa: 80 bis 250 IE/mg
- in vitro Aktivität A.P.T.T.: 10 bis 80 IE/mg
- Verhältnis $\frac{\text{in vitro Aktivität anti-Xa}}{\text{in vitro Aktivität A.P.T.T.}}$: 2 bis 10

6. Gemisch gemäß Anspruch 5, dadurch gekennzeichnet, daß es im Zustand des Natriumsalzes die folgenden Charakteristika aufweist:

- mittleres Molekulargewicht in Gewicht: 4200 Dalton
- Drehvermögen $[\alpha]_D^{20} = +33°$
- antikoagulierende Aktivität in vitro Codex: 60 IE/mg
- in vitro Aktivität anti-Xa: 140 IE/mg
- in vitro Aktivität A.P.T.T.: 33 IE/mg

7. Gemisch gemäß Anspruch 5, dadurch gekennzeichnet, daß es im Zustand des Natriumsalzes die folgenden Charakteristika aufweist:

| | |
|---|---|
| – mittleres Molekulargewicht in Gewicht: | 4100 Dalton |
| – Drehvermögen | $[\alpha]_D^{20} = +33°$ |
| – antikoagulierende Aktivität in vitro Codex: | 50 IE/mg |
| – in vitro Aktivität anti-Xa: | 130 IE/mg |
| – in vitro Aktivität A.P.T.T.: | 30 IE/mg |

8. Gemisch gemäß Anspruch 5, dadurch gekennzeichnet, daß es im Zustand des Natriumsalzes die folgenden Charakteristika aufweist:

| | |
|---|---|
| – mittleres Molekulargewicht in Gewicht: | 4500 Dalton |
| – Drehvermögen | $[\alpha]_D^{20} = +35°$ |
| – antikoagulierende Aktivität in vitro Codex: | 54 IE/mg |
| – in vitro Aktivität anti-Xa: | 110 IE/mg |
| – in vitro Aktivität A.P.T.T.: | 33 IE/mg |

9. Gemisch gemäß Anspruch 5, dadurch gekennzeichnet, daß es im Zustand des Natriumsalzes die folgenden Charakteristika aufweist:

| | |
|---|---|
| – mittleres Molekulargewicht in Gewicht: | 4000 Dalton |
| – Drehvermögen | $[\alpha]_D^{20} = +38°$ |
| – antikoagulierende Aktivität in vitro Codex: | 80 IE/mg |
| – in vitro Aktivität anti-Xa: | 159 IE/mg |
| – in vitro Aktivität A.P.T.T.: | 45 IE/mg |

10. Gemisch gemäß Anspruch 5, dadurch gekennzeichnet, daß es im Zustand des Natriumsalzes die folgenden Charakteristika aufweist:

| | |
|---|---|
| – mittleres Molekulargewicht in Gewicht: | 3800 Dalton |
| – Drehvermögen | $[\alpha]_D^{20} = +41°$ |
| – antikoagulierende Aktivität in vitro Codex: | 62 IE/mg |
| – in vitro Aktivität anti-Xa: | 159 IE/mg |
| – in vitro Aktivität A.P.T.T.: | 40 IE/mg |

11. Gemisch gemäß Anspruch 5, dadurch gekennzeichnet, daß es im Zustand des Natriumsalzes die folgenden Charakteristika aufweist:

| | |
|---|---|
| – mittleres Molekulargewicht in Gewicht: | 2800 Dalton |
| – Drehvermögen | $[\alpha]_D^{20} = +27°$ |
| – antikoagulierende Aktivität in vitro Codex: | 40 IE/mg |
| – in vitro Aktivität anti-Xa: | 90 IE/mg |
| – in vitro Aktivität A.P.T.T.: | 20 IE/mg |

12. Gemisch gemäß Anspruch 5, dadurch gekennzeichnet, daß es im Zustand des Natriumsalzes die folgenden Charakteristika aufweist:

| | |
|---|---|
| – mittleres Molekulargewicht in Gewicht: | 4000 Dalton |
| – Drehvermögen | $[\alpha]_D^{20} = +40°$ |
| – antikoagulierende Aktivität in vitro Codex: | 75 IE/mg |
| – in vitro Aktivität anti-Xa: | 140 IE/mg |
| – in vitro Aktivität A.P.T.T.: | 35 IE/mg |

13. Gemisch gemäß Anspruch 4, dadurch gekennzeichnet, daß es im Zustand des Natriumsalzes die folgenden Charakteristika aufweist:

| | |
|---|---|
| – mittleres Molekulargewicht in Gewicht: | 4000 Dalton |
| – Drehvermögen | $[\alpha]_D^{20} = +45°$ |
| – antikoagulierende Aktivität in vitro Codex: | 110 IE/mg |
| – in vitro Aktivität anti-Xa: | 170 IE/mg |
| – in vitro Aktivität A.P.T.T.: | 86 IE/mg |

14. Verfahren zur Herstellung der Gemische gemäß Anspruch 1, dadurch gekennzeichnet, daß man in wäßrigem Milieu und bei einer Temperatur von 20 bis 80°C einen in Wasser löslichen Heparinester, der

von der teilweisen oder totalen Veresterung der Säure-Carboxylgruppen des Heparins resultiert, mit einer in Wasser löslichen Mineralbase oder organischen Base zur Reaktion bringt und das so gebildete Depolymersisationsprodukt isoliert.

15. Verfahren gemäß Anspruch 14, dadurch gekennzeichnet, daß die molare Konzentration der Base in dem Milieu 0,1 bis 0,6 beträgt.

16. Verfahren zur Herstellung der Gemische gemäß Anspruch 1, dadurch gekennzeichnet, daß man einen Heparinester, der von der teilweisen oder totalen Veresterung der Säure-Carboxylgruppen des Heparins resultiert, mit einer Base in einem organischen, für diesen Ester inerten Lösungsmittel umsetzt, das so gebildete Depolymerisationsprodukt im Zustand des Alkalisalzes isoliert und es mit einer wäßrigen, mindestens 1 N Natriumhydroxidlösung bei niedriger Temperatur hydrolisiert.

17. Verfahren gemäß Anspruch 16, dadurch gekennzeichnet, daß die Reaktion des Heparinesters mit der Base bei einer Temperatur von 20°C bis 80°C durchgeführt wird.

18. Medikament, daß insbesondere zur Vorbeugung und Behandlung von Thrombosen und zur Behandlung der Hyperlipämie verwendbar ist, dadurch gekennzeichnet, daß es als aktiven Bestandteil ein Gemisch der sulfatierten Polysaccharide, wie es in jedem der Ansprüche 1 bis 13 definiert ist, enthält, wobei die Säuregruppen dieser Polysaccharide in Form von pharmazeutisch annehmbaren Salzen vorliegen.

19. Medikament gemäß Anspruch 18, dadurch gekennzeichnet, daß die Säuregruppen der sulfatierten Polysaccharide in Form des Natriumsalzes, des Calciumsalzes oder des Magnesiumsalzes vorliegen.

## Claims

1. Mixtures of sulphated polysaccharides having the general structure of the polysaccharides present in heparin, whose acid groups are in the free form or converted into a salt form, characterised in that the polysaccharides present in the said mixtures have an ethylenic double bond at one of the ends of their chain and in that, in the form of the sodium salt, the mixtures have the following characteristics:

| | |
|---|---|
| — percentage of sulphur: | 9% to 13.5% |
| — percentage of nitrogen: | 1.8% to 2.5% |
| — percentage of uronic acids: | 20% to 30% |
| — weight-average molecular weight: | 2000 to 10 000 daltons |
| — specific rotatory power in aqueous solution at 20°C: | $[\alpha]_D^{20}$ = + 25° to + 55° |

2. Mixtures according to claim 1, characterised in that the polysaccharides present in the said mixtures correspond to the formula:

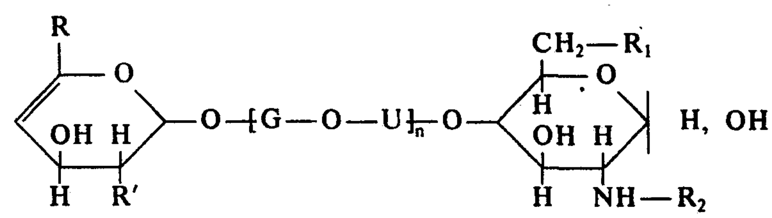

(I)

in which R designates a hydrogen atom or a carboxylic group in an acid form or converted into a salt form, R′ designates an OH group or a sulphate group, in an acid form or converted into a salt form, $R_1$ designates an OH group or a sulphate group, in an acid form or converted into a salt form, $R_2$ designates a sulphonate group, in an acid form or converted into a salt form, or an acetyl group, —O— designates an oxygen bridge, the links G designate the links of the glucosamine type present in the structure of the heparin, the links U designate the links of the uronic acid type (D-glucuronic acid, L-iduronic acid and sulphated L-iduronic acid) present in the structure of the heparin, and n is an integer form 3 to 20, the acid groups of the polysaccharides being in the free form or converted into a salt form.

3. Mixtures according to either of claims 1 and 2, characterised in that, in the form of a sodium salt, they have the following characteristics:

| | |
|---|---|
| — weight-average molecular weight: | 8000 to 10 000 daltons |
| — in vitro Codex anticoagulant activity: | 130 to 160 i.u./mg |
| — in vitro anti-Xa activity: | 130 to 180 i.u./mg |
| — in vitro A.P.T.T. activity: | 100 to 150 i.u./mg |
| — ratio of $\frac{\text{in vitro anti-Xa activity}}{\text{in vitro A.P.T.T. activity}}$: | 1 to 1.5 |

4. Mixtures according to either of claims 1 and 2, characterised in that, in the form of a sodium salt, they have the following characteristics:

| | |
|---|---|
| – weight-average molecular weight: | 3000 to 8000 daltons |
| – in vitro Codex anticoagulant activity: | 80 to 140 i.u./mg |
| – in vitro anti-Xa activity: | 120 to 250 i.u./mg |
| – in vitro A.P.T.T. activity: | 80 to 120 i.u./mg |
| – ratio of $\frac{\text{in vitro anti-Xa activity}}{\text{in vitro A.P.T.T. activity}}$: | 1.4 to 3 |

5. Mixtures according to either of claims 1 and 2, characterised in that, in the form of a sodium salt, they have the following characteristics:

| | |
|---|---|
| – weight-average molecular weight: | 2000 to 7000 daltons |
| – in vitro Codex anticoagulant activity: | 10 to 80 i.u./mg |
| – in vitro anti-Xa activity: | 80 to 250 i.u./mg |
| – in vitro A.P.T.T. activity: | 10 to 80 i.u./mg |
| – ratio of $\frac{\text{in vitro anti-Xa activity}}{\text{in vitro A.P.T.T. activity}}$: | 2 to 10 |

6. A mixture according to claim 5, characterised in that, in the form of a sodium salt, it has the following characteristics:

| | |
|---|---|
| – weight-average molecular weight: | 4200 daltons |
| – rotatory power | $[\alpha]_D^{20} = +33°$ |
| – in vitro Codex anticoagulant activity: | 60 i.u./mg |
| – in vitro anti-Xa activity: | 140 i.u./mg |
| – in vitro A.P.T.T. activity: | 33 i.u./mg |

7. A mixture according to claim 5, characterised in that, in the form of a sodium salt, it has the following characteristics:

| | |
|---|---|
| – weight-average molecular weight: | 4100 daltons |
| – rotatory power | $[\alpha]_D^{20} = +33°$ |
| – in vitro Codex anticoagulant activity: | 50 i.u./mg |
| – in vitro anti-Xa activity: | 130 i.u./mg |
| – in vitro A.P.T.T. activity: | 30 i.u./mg |

8. A mixture according to claim 5, characterised in that, in the form of a sodium salt, it has the following characteristics:

| | |
|---|---|
| – weight-average molecular weight: | 4500 daltons |
| – rotatory power | $[\alpha]_D^{20} = +35°$ |
| – in vitro Codex anticoagulant activity: | 54 i.u./mg |
| – in vitro anti-Xa activity: | 110 i.u./mg |
| – in vitro A.P.T.T. activity: | 33 i.u./mg |

9. A mixture according to claim 5, characterised in that, in the form of a sodium salt, it has the following characteristics:

| | |
|---|---|
| – weight-average molecular weight: | 4000 daltons |
| – rotatory power | $[\alpha]_D^{20} = +38°$ |
| – in vitro Codex anticoagulant activity: | 80 i.u./mg |
| – in vitro anti-Xa activity: | 159 i.u./mg |
| – in vitro A.P.T.T. activity: | 45 i.u./mg |

10. A mixture according to claim 5, characterised in that, in the form of a sodium salt, it has the following characteristics:

| | |
|---|---|
| – weight-average molecular weight: | 3800 daltons |
| – rotatory power | $[\alpha]_D^{20} = +41°$ |
| – in vitro Codex anticoagulant activity: | 62 i.u./mg |

- in vitro anti-Xa activity: 159 i.u./mg
- in vitro A.P.T.T. activity: 40 i.u./mg

11. A mixture according to claim 5, characterised in that, in the form of a sodium salt, it has the following characteristics:

- weight-average molecular weight: 2800 daltons
- rotatory power $[\alpha]_D^{20} = +27°$
- in vitro Codex anticoagulant activity: 40 i.u./mg
- in vitro anti-Xa activity: 90 i.u./mg
- in vitro A.P.T.T. activity: 20 i.u./mg

12. A mixture according to claim 5, characterised in that, in the form of a sodium salt, it has the following characteristics:

- weight-average molecular weight: 4000 daltons
- rotatory power $[\alpha]_D^{20} = +40°$
- in vitro Codex anticoagulant activity: 75 i.u./mg
- in vitro anti-Xa activity: 140 i.u./mg
- in vitro A.P.T.T. activity: 35 i.u./mg

13. A mixture according to claim 4, characterised in that, in the form of a sodium salt, it has the following characteristics:

- weight-average molecular weight: 4000 daltons
- rotatory power $[\alpha]_D^{20} = +45°$
- in vitro Codex anticoagulant activity: 110 i.u./mg
- in vitro anti-Xa activity: 170 i.u./mg
- in vitro A.P.T.T. activity: 86 i.u./mg

14. A process for the preparation of the mixtures according to claim 1, characterised in that a water-soluble ester of heparin, produced by partial or complete esterification of the heparin carboxylic acid groups is reacted with an inorganic base or a water-soluble organic base in an aqueous medium and at a temperature from 20°C to 80°C, and the depolymerisation product thus produced is separated.

15. A process according to claim 14, characterised in that the molar concentration of the base in the medium is 0.1 to 0.6.

16. A process for the preparation of the mixtures according to claim 1, characterised in that an ester of heparin produced by partial or complete esterification of the heparin carboxylic acid groups is reacted with a base, in an organic solvent unreactive towards the said ester, the depolymerisation product thus produced is separated in the form of an alkali metal salt and is hydrolysed at a low temperature with an aqueous solution of sodium hydroxide whose normality is at least 1 N.

17. A process according to claim 16, characterised in that the reaction of the heparin ester with the base is carried out at a temperature of 20°C to 80°C.

18. A medication which may be employed in particular for the prevention and the treatment of thrombosis and for the treatment of hyperlipemia, characterised in that it comprises as an active ingredient a mixture of sulphated polysaccharides such as specified in each of the claims 1 to 13, the acid groups of the said polysaccharides being in the form of pharmaceutically acceptable salts.

19. A medication according to claim 18, characterised in that the acid groups of the sulphated polysaccharides are in the form of a sodium salt, a calcium salt or a magnesium salt.